# EUROPÄISCHE PATENTSCHRIFT

(11) **EP 1 059 070 B1**
(45) Veröffentlichungstag und Bekanntmachung des Hinweises auf die Patenterteilung: **06.04.2005**
(21) Anmeldenummer: 00111871.0
(22) Anmeldetag: 09.06.2000
(51) Int. Cl.: A61F 2/36, A61F 2/46

(54) **Prothesenschaft**
Prosthesis shaft
Tige de prothèse

(30) Priorität: 10.06.1999 DE 19926391
(43) Veröffentlichungstag der Anmeldung: 13.12.2000
(73) Patentinhaber: Aesculap AG & Co. KG, 78532 Tuttlingen (DE)
(72) Erfinder: Herb, Markus, 86438 Kissing (DE); Reu, Gerhard, 78532 Tuttlingen (DE)
(74) Vertreter: HOEGER, STELLRECHT & PARTNER Patentanwälte

(56) Entgegenhaltungen:
- EP-A- 0 198 163
- EP-A- 0 436 317
- EP-A- 0 634 154
- EP-A- 0 649 642
- EP-A- 0 792 628
- EP-A- 0 888 759
- EP-A- 1 004 283
- EP-A- 1 004 284
- WO-A-93/08770
- WO-A-96/06576
- DE-U- 29 910 113
- DE-U- 29 918 669
- FR-A- 2 730 628
- GB-A- 2 223 172
- US-A- 2 679 245

## Beschreibung

Die Erfindung betrifft einen modular aufgebauter Prothesenschaft mit konischen Verbindungsflächen zur Festlegung benachbarter Teile des Prothesenschaftes aneinander mit einem eine zapfenförmige Verlängerung tragenden ersten Teil des Prothesenschaftes und mit einem eine Durchgangsbohrung zur Aufnahme der Verlängerung aufweisenden benachbarten zweiten Teil des Prothesenschaftes, wobei zwischen der Verlängerung einerseits und der Innenwand der Durchgangsbohrung andererseits ein sich in Richtung auf das erste Teil keilförmig verengender Ringspalt angeordnet ist.

Insbesondere bei der zementfreien Implantation von Hüftgelenkprothesen ist es notwendig, daß die Implantate möglichst genau mit der Markraumgeometrie des Patienten übereinstimmen, dies gilt insbesondere für den Schaft der Femurprothese. In vielen Fällen wird dieser Schaft aus Einzelteilen zusammengesetzt, also modular aufgebaut, und dann ist es notwendig, die Einzelteile der Schaftprothese dauerhaft zu verbinden.

Es ist bekannt, Einzelteile einer modular aufgebauten Schaftprothese mit einem konusförmigen Zapfen zu versehen, der in eine konusförmige, komplementäre Ausnehmung des benachbarten Teiles klemmend eingeschoben werden kann. Die Verbindung erfolgt dabei entweder durch Selbsthemmung oder auch unter Mithilfe eines Zugankers, der durch geeignete Spannmittel die beiden Teile gegeneinander spannt.

Ein gattungsgemäßer Prothesenschaft ist bekannt aus der EP 0 634 154 A1. Zum Verspannen der beiden Prothesenteile trägt ein auf die zapfenförmige Verlängerung aufschraubbares Spannelement einen konischen Abschnitt, dessen Außenwand sich an die Innenwand der Durchgangsbohrung anlegt und beim Aufschrauben des Spannelementes auf das Gewinde der zapfenförmigen Verlängerung eine Verklemmung hervorruft. Das Spannelement weist über seine ganze Länge ein Innengewinde auf und muß so stabil ausgeführt sein, daß die Spannkräfte aufgebracht werden. Dadurch ist ein retativ voluminöser Aufbau notwendig.

Ausgehend von diesem Stand der Technik liegt der Erfindung die Aufgabe zugrunde, auch unter erschwerten Zugangsbedingungen zuverlässig eine Verbindung von zwei benachbarten Teilendes Prothesenschaftes zu erreichen, insbesondere dann, wenn aufgrund des zur Verfügung stehenden Platzes der Ringspalt sehr schmal ausgebildete werden muß.

Diese Aufgabe wird bei einem Prothesenschaft der eingangs beschriebenen Art erfindungsgemäß dadurch gelöst, daß in den Ringspalt eine komplementär ausgebildete Klemmhülse eintaucht, und daß an der Verlängerung ein in Richtung auf das erste Teil bewegbares Spannelement gehalten ist, welches an der Klemmhülse anliegt und diese im gespannten Zustand in den Ringspalt eindrückt.

Die Festlegung der beiden Teile des Prothesenschaftes aneinander erfolgt also in diesem Falle durch Klemmung, die durch das kräftige Eindrücken der Klemmhülse in den keilförmigen Ringspalt erzeugt wird. Die Klemmhülse ist also gegenüber der zapfenförmigen Verlängerung frei verschiebbar und wird durch das Spannelement in den keilförmigen Ringspalt eingeschoben, dabei können Ringspalt und Klemmhülse sehr dünnwandig ausgebildet sein, da die Klemmhülse kein Gewinde zum Aufschrauben auf die Verlängerung benötigt, sondern durch ein separates Spannelement in den Ringspalt eingeschoben wird.

Die zapfenförmige Verlängerung kann im Bereich des Ringspaltes insbesondere kreiszylindrisch ausgebildet sein, so daß der Ringspalt dadurch gebildet wird, daß die Innenwand der Durchgangsbohrung sich in Richtung auf das erste Teil konisch verjüngt.

Bei einer bevorzugten Ausführungsform weist die zapfenförmige Verlängerung auf ihrer dem ersten Teil zugewandten Seite einen sich in Richtung zum Ringspalt hin konisch verjüngenden Abschnitt auf, der an einem komplementären, sich in Richtung zum ersten Teil konisch erweiternden Abschnitt der Innenwand der Durchgangsbohrung anliegt. Auf diese Weise wirken zwei konische Anlageflächen gemeinsam bei der Festlegung der beiden Teile mit, wobei diese konischen Anlageflächen sich in entgegengesetzter Richtung öffnen und wobei an einer dieser konischen Anlageflächen die eingepreßte Klemmhülse anliegt.

Die Durchgangsbohrung kann bei einem ersten Ausführungsbeispiel selbst so ausgebildet sein, daß sie diese konischen Anlageflächen oder zumindest eine der beiden konischen Anlageflächen aufweist, so daß die Klemmhülse unmittelbar zwischen die zapfenförmige Verlängerung des einen Teils einerseits und die konische Anlagefläche des zweiten Teils andererseits eintaucht.

Bei einem ebenfalls bevorzugten Ausführungsbeispiel kann jedoch vorgesehen sein, daß die Innenwand der Durchgangsbohrung im Bereich der zapfenförmigen Verlängerung von einer diese umgebenden, an der Außenseite kreiszylindrischen Hülse gebildet wird, die in die ebenfalls kreiszylindrische Durchgangsbohrung eintaucht. Die konische Anlagefläche oder die konischen Anlageflächen des zweiten Teils werden also nicht durch dieses selbst ausgebildet, sondern durch eine dünne Hülse, die in die zylindrische Durchgangsbohrung eingeschoben ist und die somit die Innenwand der Durchgangsbohrung in dem die Verlängerung des ersten Teils umgebenden Bereich verengt. Die Innenwand der Hülse bildet in diesem Bereich die Innenwand der Durchgangsbohrung aus, die aber nicht einstückig mit dem zweiten Teil des Prothesenschaftes ausgebildet ist.

Die Festlegung der beiden Teile des Prothesenschaftes aneinander erfolgt bei diesem Ausführungsbeispiel dadurch, daß durch das Einschieben der Klemmhülse in den keilförmigen Ringspalt die Verlängerung gegenüber der Innenwand der Hülse verklemmt wird, außerdem wird die Hülse in diesem Bereich aufgeweitet und wird klemmend gegen die zylindrische Durchgangsbohrung des zweiten Teils des Prothesenschaftes gepreßt.

Dabei ist es vorteilhaft, wenn die Hülse zumindest im Bereich des Ringspaltes vom Rand ausgehende, achsparallele Einschnitte aufweist, dies erleichtert das Auseinanderdrücken der Hülse im Bereich des Ringspaltes.

Grundsätzlich ist denkbar, das Spannelement als Spannschraube mit Außengewinde auszubilden. Gemäß einer bevorzugten Ausführungsform ist das Spannelement eine Spannmutter mit Innengewinde, die auf einen Gewindezapfen am Ende der Verlängerung aufgeschraubt ist.

Gemäß einer bevorzugten Ausführungsform ist dabei vorgesehen, daß die Spannmutter an ihrem dem Ringspalt abgewandten Ende einen Eindrehabschnitt mit Anlageflächen für ein Eindrehwerkzeug trägt, der über eine Sollbruchstelle mit dem übrigen Teil der Spannmutter verbunden ist. Eine solche Ausgestaltung ermöglicht es, die Spannmutter mit einem genau definierten Drehmoment anzuziehen. Beim Erreichen dieses maximalen Drehmomentes schert der Eindrehabschnitt im Bereich der Sollbruchstelle ab, und damit ist sichergestellt, daß auch die Klemmhülse mit genau vorbestimmter Axialkraft in den Ringspalt gepreßt wird.

Die Anlageflächen können dabei durch einen Innenmehrkant gebildet werden.

Es ist günstig, wenn die Sollbruchstelle in Höhe des Endes der Durchgangsbohrung angeordnet ist, so daß die Durchgangsbohrung durch den verbleibenden Teil der Spannmutter bündig abgeschlossen wird.

Bei einer bevorzugten Ausführungsform ist vorgesehen, daß in der Spannmutter ein Anlagenflächen für ein Ausdrehwerkzeug aufweisender Innenmehrkant angeordnet ist, der nach Entfernung des Eindrehabschnittes zugänglich ist. Dies ermöglicht das nachträgliche Lösen der miteinander verbundenen Teile des Prothesenschaftes.

Günstig ist es dabei, wenn der Innenmehrkant für das Ausdrehwerkzeug durch eine Verlängerung des Innenmehrkantes im Eindrehabschnitt gebildet wird.

Bei einer bevorzugten Ausführungsform ist vorgesehen, daß zwischen dem Eindrehabschnitt und dem übrigen Teil der Spannmutter eine kerbenförmige Aufnahme für den Eindrehabschnitt zwischen sich aufnehmende Halteelemente eines Eindrehwerkzeuges angeordnet sind. Ein solches Eindrehwerkzeug kann somit mittels dieser Haltearme den Eindrehabschnitt zwischen sich halten, der nach dem Abscheren auf diese Weise nicht verloren geht, sondern mit dem Eindrehwerkzeug aus dem Operationsbereich entfernt werden kann.

Zum Eindrehen der Spannmutter kann verteilhaft ein Eindrehwerkzeug verwendet werden, bei welchem vorgesehen ist, daß es einen Anschlag aufweist, der die Eintauchtiefe von an den Anlageflächen der Spannmutter anliegenden Mitnahmeflächen auf den Eindrehabschnitt begrenzt. Dies stellt sicher, daß auch bei einer in den übrigen Teil der Spannmutter verlängerten Innenmehrkantausnehmung das Eindrehwerkzeug drehfest nur mit dem Eindrehabschnitt verbunden wird, so daß die Funktion der Sollbruchstelle gewährleistet ist.

Es ist auch vorteilhaft, wenn ein solches Eindrehwerkzeug an seinem Ende fingerförmige Halteelemente trägt, die den Eindrehabschnitt der Spannmutter zwischen sich festhalten, wenn dieser bei Erreichen des maximalen Drehmomentes von der übrigen Spannmutter getrennt wird.

Die fingerförmigen Halteelemente können dabei an ihren freien Enden Vorsprünge tragen, die den Eindrehabschnitt der Spannmutter umgreifen, so daß die Sicherheit erhöht wird, mit der der Eindrehabschnitt am Eindrehwerkzeug festgelegt wird.

Die nachfolgende Beschreibung bevorzugter Ausführungsformen der Erfindung dient im Zusammenhang mit der Zeichnung der näheren Erläuterung. Es zeigen:
- Figur 1:: eine Teilseitenansicht eines teilweise aufgebrochen dargestellten Prothesenschaftes mit aufgesetztem Eindrehwerkzeug;
- Figur 2:: eine Ansicht ähnlich Figur 1 nach dem Verspannen der Einzelteile des Prothesenschaftes und
- Figur 3:: eine Ansicht ähnlich Figur 2 bei einem abgewandelten Ausführungsbeispiel eines Prothesenschaftes.

Der in der Zeichnung dargestellte Prothesenschaft ist im wesentlichen aus zwei Teilen aufgebaut, nämlich einem Stiel 1 und einem Oberteil 2, welches einen seitlich abstehenden Verbindungszapfen 3 für eine in der Zeichnung nicht dargestellte Gelenkkugel trägt.

Zur Verbindung des Stieles 1 mit dem Oberteil 2 ist das Oberteil 2 mit einer zylindrischen Durchgangsbohrung 4 versehen, die dieses vollständig durchsetzt. Der Stiel 1 trägt an seiner Oberseite eine zapfenförmige Verlängerung 5 mit einem ersten, konisch ausgebildeten Abschnitt 6, dessen Durchmesser in Richtung auf das freie Ende der Verlängerung 5 abnimmt, mit einem sich an den konischen Abschnitt 6 anschließenden zylindrischen Abschnitt 7 und mit einem Außengewindezapfen 8.

Die Verlängerung 5 wird umgeben von einer dünnwandigen Hülse 9 mit einer kreiszylindrischen Außenfläche 10 und mit einer ebenfalls kreiszylindrischen Innenfläche 11, die sich jeweils zu den Enden der Hülse 9 hin konisch erweitert und dort konische Anlageflächen 12 bzw. 13 ausbildet. Die untere konische Anlagefläche 13 ist dabei komplementär ausgebildet zu dem konischen Abschnitt 6 der Verlängerung 5, die obere konische Anlagefläche 12 hingegen entfernt sich zum Rand der Hülse 9 hin immer mehr vom zylindrischen Abschnitt der Verlängerung 5 und bildet mit dieser gemeinsam einen keilförmigen Ringspalt 14 aus, der sich zum Rand der Hülse hin erweitert.

In diesen Ringspalt 14 ist eine der Querschnittsform des Ringspaltes 14 komplementäre Klemmhülse 15 eingeschoben, die über den Rand der Hülse 9 aus dem Ringspalt 14 hervorsteht.

Auf den Außengewindezapfen 8 ist eine mit einem Innengewinde 16 versehene Spannmutter 17 aufgeschraubt, die in die Durchgangsbohrung 4 eintaucht und diese ausfüllt.

Diese Spannmutter 17 weist einen unteren Spannbereich 18 auf und einen oberen Eindrehbereich 19, die durch eine umlaufende Ringkerbe 20 voneinander getrennt sind. Im Bereich de Ringkerbe 20 sind der Spannbereich 18 und der Eindrehbereich 19 nur durch einen schmalen Materialsteg 21 miteinander verbunden, so daß in diesem Bereich eine Sollbruchstelle entsteht. In axialer Ausrichtung mit dem Innengewinde 16 ist an der Oberseite der Spannmutter 17 ein Innenmehrkant 22 in die Spannmutter 17 eingearbeitet, dessen Innenflächen Anlageflächen für Ein- und Ausdrehwerkzeuge bilden. Der Innenmehrkant 22 ist so tief, daß er den Eindrehbereich 19 vollständig durchsetzt und teilweise in den Spannbereich 18 eintaucht.

Zur Verbindung des Stieles 1 mit dem Oberteil 2 wird der Stiel 1 mit der Verlängerung 5 in die Durchgangsbohrung 4 eingeschoben, in den Zwischenraum zwischen Verlängerung 5 und Durchgangsbohrung 4 wird die Hülse 9 eingeführt, wobei es grundsätzlich auch möglich ist, die Hülse 9 von Anfang an auf die Verlängerung 5 aufzuschieben und den Stiel 1 erst dann in die Durchgangsbohrung 4 einzuführen.

In den Ringspalt 14 wird dann die Klemmhülse 15 eingelegt, und die Spannmutter 17 wird auf den Außengewindezapfen 8 aufgeschraubt, wobei sich die Unterkante der Spannmutter 17 an die Oberkante der Klemmhülse 15 anlegt und diese beim weiteren Aufschrauben in den Ringspalt 14 einpreßt. Durch diese Pressung wird der Ringspalt 14 aufgeweitet, und dies führt einmal zu einer Verklemmung der Verlängerung 5 relativ zur Hülse 9 und zum anderen zu einer Aufweitung der Hülse 9, die sich dadurch gegenüber der Durchgangsbohrung 4 verklemmt. Außerdem wird die Verlängerung 5 dadurch kräftig in die Hülse 9 hineingezogen, so daß der konische Abschnitt 6 der Verlängerung 5 kräftig gegen die konische Anlagefläche 12 der Hülse 9 gepreßt wird, die dadurch ebenfalls aufgeweitet und gegen die Durchgangsbohrung 4 gepreßt wird. Insgesamt führt diese doppelte Verklemmung zu einer sicheren axialen Festlegung der Verlängerung 5 in der Durchgangsbohrung 4.

Die Spannmutter 17 wird mit einem definierten Drehmoment eingedreht. Dies kann beispielsweise dadurch erfolgen, daß ein an sich bekannter Drehmomentenschlüssel verwendet wird.

Bei der speziellen Ausgestaltung der Spannmutter, die oben beschrieben worden ist, ist dies jedoch nicht unbedingt notwendig, da beim Erreichen eines maximalen Drehmomentes die Sollbruchstelle im Bereich des Materialsteges 21 abgeschert wird, so daß ein weiteres Eindrehen unmöglich wird.

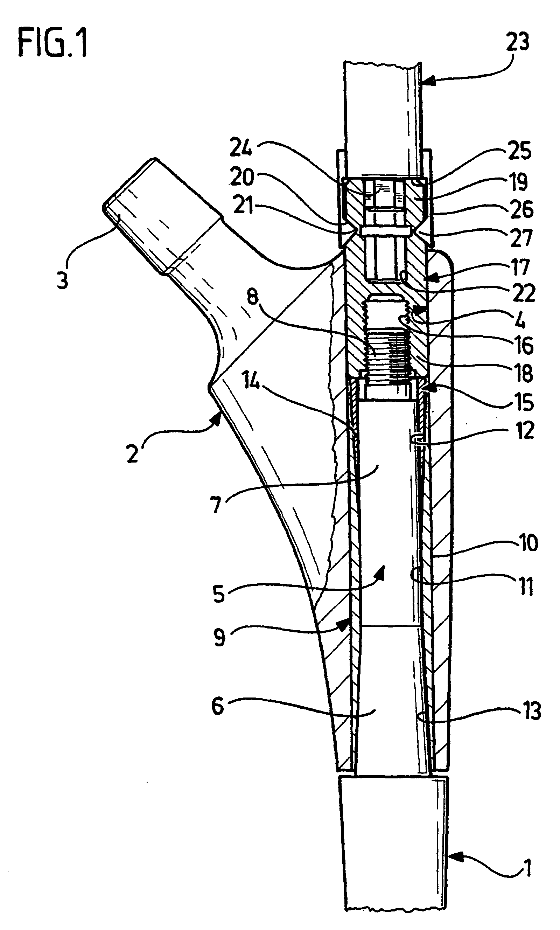

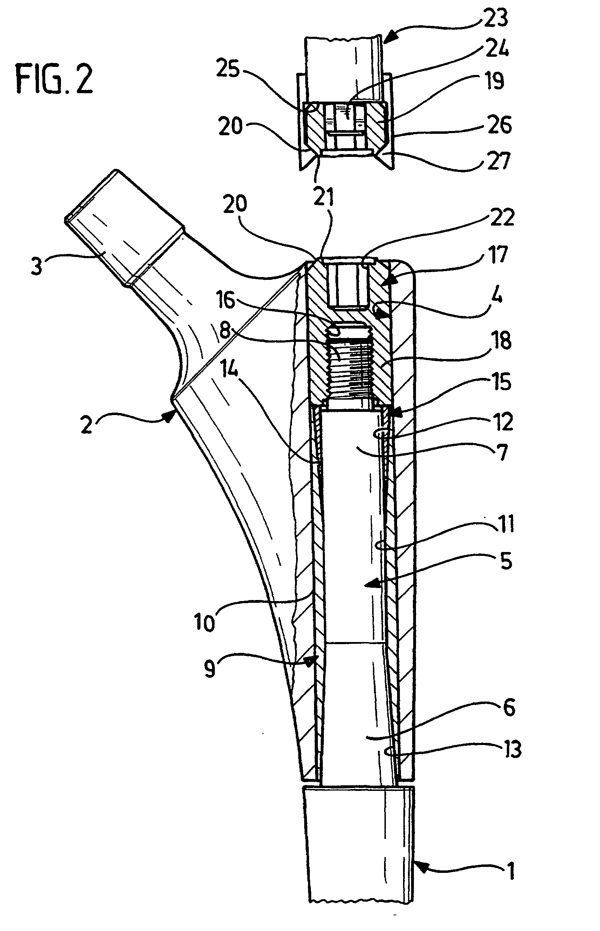

Besonders vorteilhaft für das Eindrehen der Spannmutter 17 ist dabei ein Eindrehwerkzeug 23, wie es in den Figuren 1 und 2 dargestellt ist. Dieses weist einen Eindrehdorn 24 auf, der komplementär zu dem Innenmehrkant 22 ausgebildet ist und dessen Länge nur so groß gewählt ist, daß er in den Eindrehbereich 19 eintaucht, nicht aber bis in den Spannbereich 18 reicht. Dies wird beispielsweise dadurch sichergestellt, daß sich das Eindrehwerkzeug 23 stufenförmig erweitert und mit der dadurch ausgebildeten Stufe 25 an der Oberseite des Eindrehbereiches 19 auf der Spannmutter 17 aufsitzt.

Das Eindrehwerkzeug 23 trägt an seinem Ende mehrere über den Umfang verteilte, achsparallele Haltefinger 26, die an ihrem freien Ende nach innen gerichtete Vorsprünge 27 aufweisen. Setzt man das Eindrehwerkzeug 23 mit dem Eindrehdorn 24 so an die Spannmutter 17 an, daß der Eindrehdorn 24 in den Innenmehrkant 22 des Eindrehbereiches 19 eintaucht, so legen sich die Haltefinger 26 an die Außenseite des Eindrehbereichs 19 an, die Vorsprünge 27 tauchen in die Ringkerbe 20 ein.

Beim Erreichen des maximalen Eindrehmomentes bricht die Sollbruchstelle, der dadurch von der übrigen Spannmutter 17 getrennte Eindrehbereich 19 bleibt nach der Abtrennung mit dem Eindrehwerkzeug 23 verbunden, da die Haltefinger 26 mit den in die Ringkerbe 20 eingreifenden Vorsprüngen 27 diesen Eindrehbereich 19 festhalten. Dadurch ist sichergestellt, daß der Eindrehbereich 19 nach dem Abtrennen nicht im Operationsbereich verlorengeht.

Nach dem Verbinden der beiden Teile des Prothesenschaftes in der beschriebenen Weise wird die Durchgangsbohrung 4 am oberen Ende durch den eingeschraubten Spannbereich 18 der Spannmutter 17 bündig abgeschlossen, die Spannmutter 17 hat somit gleichzeitig die Funktion eines Verschlußstopfens.

Durch den in den Spannbereich 18 eintauchenden Teil des Innenmehrkantes 22 ist es jederzeit möglich, mit einem geeigneten Ausdrehwerkzeug die Spannmutter 17 wieder vom Außengewindezapfen 8 abzuschrauben und damit die beschriebene Verbindung zu lösen.

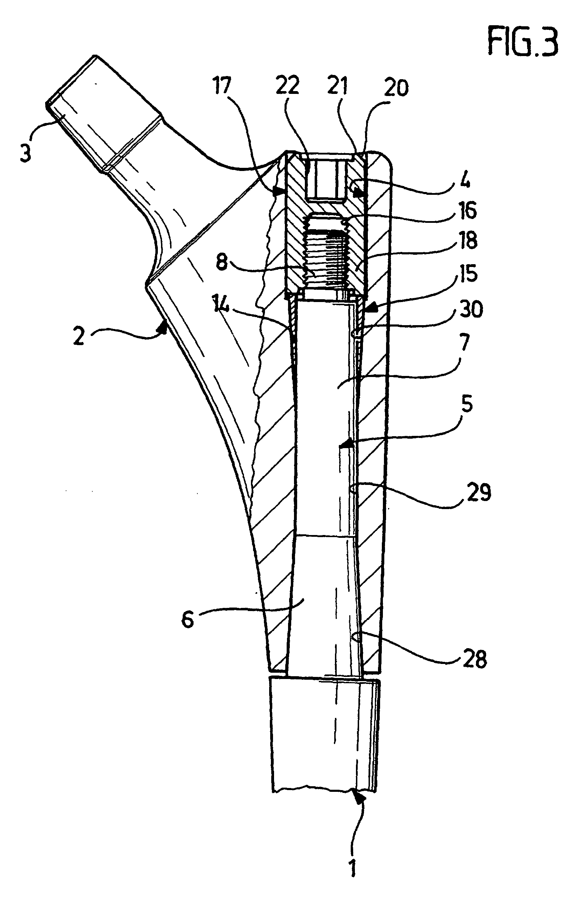

Bei dem Ausführungsbeispiel der Figur 3 ist ein sehr ähnlicher Aufbau gewählt, einander entsprechende Teile tragen daher dieselben Bezugszeichen. Das Ausführungsbeispiel der Figur 3 unterscheidet sich von dem der Figuren 1 und 2 lediglich dadurch, daß die Hülse 9 fehlt und daß statt dessen die Innenwand der Durchgangsbohrung 4 nicht kreiszylindrisch ausgebildet ist sondern im Bereich der Verlängerung 5 entsprechend der Innenwand der Hülse 9 beim Ausführungsbeispiel der Figuren 1 und 2 konturiert ist. Die Innenwand der Durchgangsbohrung 4 weist also eine Durchgangsbohrung auf, in der sich von unten nach oben ein sich konisch verengender Abschnitt 28, ein kreiszylindrischer Abschnitt 29, ein sich konisch erweiternder Abschnitt 30 und ein zylindrischer Abschnitt 31 mit größerem Außendurchmesser aneinander anschließen. Der konische Abschnitt 28 liegt dabei dem konischen Abschnitt 6 der Verlängerung 5 gegenüber, der konische Abschnitt 30 bildet zusammen mit dem zylindrischen Abschnitt 7 der Verlängerung 5 den Ringspalt 14 aus.

Montage und Demontage des Prothesenschafts der Figur 3 erfolgen in gleicher Weise wie bei dem Prothesenschaft der Figuren 1 und 2.

## Patentansprüche

1. Modular aufgebauter Prothesenschaft mit konischen Verbindungsflächen zur Festlegung benachbarter Teile des Prothesenschaftes aneinander mit einem eine zapfenförmige Verlängerung (5) tragenden ersten Teil (1) des Prothesenschaftes und mit einem eine Durchgangsbohrung (4) zur Aufnahme der Verlängerung (5) aufweisenden benachbarten zweiten Teil (2) des Prothesenschaftes, wobei zwischen der Verlängerung (5) einerseits und der Innenwand (12; 30) der Durchgangsbohrung (4) andererseits ein sich in Richtung auf das erste Teil (1) keilförmig verengender Ringspalt (14) angeordnet ist, **dadurch gekennzeichnet, daß** in den Ringspalt eine komplementär ausgebildete Klemmhülse (15) eintaucht, und daß an der Verlängerung (5) ein in Richtung auf das erste Teil (1) bewegbares Spannelement (17) gehalten ist, welches an der Klemmhülse (15) anliegt und diese im gespannten Zustand in den Ringspalt (14) eindrückt.

2. Prothesenschaft nach Anspruch 1, **dadurch gekennzeichnet, daß** die zapfenförmige Verlängerung (5) im Bereich des Ringspaltes (14) kreiszylindrisch ausgebildet ist.

3. Prothesenschaft nach einem der Ansprüche 1 oder 2, **dadurch gekennzeichnet, daß** die zapfenförmige Verlängerung (5) auf ihrer dem ersten Teil (1) zugewandten Seite einen sich in Richtung zum Ringspalt (14) hin konisch verjüngenden Abschnitt (6) aufweist, der an einem komplementären, sich in Richtung zum ersten Teil (1) konisch erweiternden Abschnitt (13; 28) der Durchgangsbohrung (4) anliegt.

4. Prothesenschaft nach einem der voranstehenden Ansprüche, **dadurch gekennzeichnet, daß** die Innenwand (11, 12, 13) der Durchgangsbohrung (4) im Bereich der zapfenförmigen Verlängerung (5) von einer diese umgebenden, an der Außenseite (10) kreiszylindrischen Hülse (9) gebildet wird, die in die ebenfalls kreiszylindrische Durchgangsbohrung (4) eintaucht.

5. Prothesenschaft nach Anspruch 4, **dadurch gekennzeichnet, daß** die Hülse (9) zumindest im Bereich des Ringspaltes (14) vom Rand ausgehende achsparallele Einschnitte aufweist.

6. Prothesenschaft nach einem der voranstehenden Ansprüche, **dadurch gekennzeichnet, daß** das Spannelement eine Spannmutter (17) mit Innengewinde (16) ist, die auf einen Gewindezapfen (8) am Ende der Verlängerung (5) aufgeschraubt ist.

7. Prothesenschaft nach Anspruch 6, **dadurch gekennzeichnet, daß** die Spannmutter (17) an ihrem dem Ringspalt (14) abgewandten Ende einen Eindrehabschnitt (19) mit Anlageflächen (22) für ein Eindrehwerkzeug (23) trägt, der über eine Sollbruchstelle (21) mit dem übrigen Teil der Spannmutter (17) verbunden ist.

8. Prothesenschaft nach Anspruch 7, **dadurch gekennzeichnet, daß** die Anlageflächen durch einen Innenmehrkant (22) gebildet werden.

9. Prothesenschaft nach Anspruch 7 oder 8, **dadurch gekennzeichnet, daß** die Sollbruchstelle (21) in Höhe des Endes der Durchgangsbohrung (4) angeordnet ist.

10. Prothesenschaft nach einem der Ansprüche 7 bis 9, **dadurch gekennzeichnet, daß** in der Spannmutter (17) ein Anlageflächen für ein Ausdrehwerkzeug aufweisender Innenmehrkant (22) angeordnet ist, der nach Entfernung des Eindrehabschnittes (19) zugänglich ist.

11. Prothesenschaft nach einem der Ansprüche 8 und 10, **dadurch gekennzeichnet, daß** der Innenmehrkant (22) für das Ausdrehwerkzeug durch eine Verlängerung des Innenmehrkants (22) im Eindrehabschnitt (19) gebildet wird.

12. Prothesenschaft nach einem der Ansprüche 7 bis 11, **dadurch gekennzeichnet, daß** zwischen dem-Eindrehabschnitt (19) und dem übrigen Teil (18) der Spannmutter (17) eine kerbenförmige Aufnahme (20) für den Eindrehabschnitt (19) zwischen sich aufnehmende Halteelemente (26) eines Eindrehwerkzeuges (23) angeordnet sind.

## Claims

1. A prosthetic shaft of modular construction with conical connecting surfaces for fastening adjoining parts of the prosthetic shaft to one another, having a first part (1) of the prosthetic shaft bearing a peg-like extension (5) and having an adjoining second part (2) of the prosthetic shaft, which second part (2) has a throughbore (4) for accommodating the extension (5), wherein an annular cleft (14) narrowing in the form of a wedge in the direction of the first part (1) is arranged between the extension (5) on the one hand and the inside wall (12; 30) of the throughbore (4) on the other, **characterised in that** a clamping sleeve (15) of complementary design penetrates the annular cleft and **in that** a tensioning member (17) movable in the direction of the first part (1) is held on the extension (5), which tensioning member lies against the clamping sleeve (15) and presses it into the annular cleft (14) in the tensioned state.

2. A prosthetic shaft according to Claim 1, **characterised in that**, in the region of the annular cleft (14), the peg-like extension (5) is shaped like a circular cylinder.

3. A prosthetic shaft according to any one of Claims 1 or 2, **characterised in that**, on its side facing towards the first part (1), the peg-like extension (5) has a portion (6) which narrows conically in the direction of the annular cleft (14), which portion (6) lies against a complementary portion (13; 28) of the throughbore (4), which portion (13; 28) widens conically in the direction of the first part (1).

4. A prosthetic shaft according to any one of the preceding claims, **characterised in that**, in the region of the peg-like extension (5), the inner wall (11, 12, 13) of the throughbore (4) is formed by a sleeve (9) surrounding the said extension (5), which sleeve is circular-cylindrical on the outside (10) and penetrates the similarly circular-cylindrical throughbore (4).

5. A prosthetic shaft according to Claim 4, **characterised in that**, at least in the region of the annular cleft (14), the sleeve (9) has axially parallel notches starting from the margin.

6. A prosthetic shaft according to any one of the preceding claims, **characterised in that** the tensioning member is a tensioning nut (17) with an internal thread (16), which nut (17) is screwed on to a threaded peg (8) at the end of the extension (5).

7. A prosthetic shaft according to Claim 6, **characterised in that** the tensioning nut (17) bears, at its end facing away from the annular cleft (14), a screw-in portion (19) with bearing surfaces (22) for a screwing tool (23), which screw-in portion is connected to the remainder of the tensioning nut (17) via a break-point (21).

8. A prosthetic shaft according to Claim 7, **characterised in that** the bearing surfaces are formed by an internal polygon (22).

9. A prosthetic shaft according to Claim 7 or 8, **characterised in that** the break-point (21) is positioned at the height of the end of the throughbore (4).

10. A prosthetic shaft according to any one of Claims 7 to 9, **characterised in that** an internal polygon (22) having bearing surfaces for an unscrewing tool is arranged within the tensioning nut (17), which internal polygon is accessible following removal of the screw-in portion (19).

11. A prosthetic shaft according to either Claim 8 or 10, **characterised in that** the internal polygon (22) for the unscrewing tool is formed by an extension of the internal polygon (22) in the screw-in portion (19).

12. A prosthetic shaft according to any one of Claims 7 to 11, **characterised in that** a notch-like receiver (20) for the screw-in portion (19) between mutually-accommodating retaining members (26) of a screwing tool (23) is arranged between the screw-in portion (19) and the remaining part (18) of the tensioning nut (17).

## Revendications

1. Tige de prothèse modulaire avec des surfaces d'assemblage coniques permettant de fixer des parties contiguës de la tige de prothèse l'une contre l'autre, comportant une première partie de tige (1) portant un prolongement (5) en forme de pivot et une deuxième partie de tige (2) contiguë munie d'une forure débouchante (4) destinée à recevoir le prolongement (5), une fente annulaire (14), rétrécie en cône en direction de la première partie (1), étant disposée entre le prolongement (5), d'une part, et la paroi intérieure (12 ; 30) de la forure débouchante (4), d'autre part, **caractérisée en ce qu'**une douille de blocage (15) de forme complémentaire est logée dans la fente annulaire (14) et **en ce qu'**un élément de serrage (17) mobile en direction de la première partie (1) est maintenu sur le prolongement,(5) de manière à venir en appui contre la douille de blocage (15) et à pousser celle-ci en position serrée dans la fente annulaire (14).

2. Tige de prothèse selon la revendication 1, **caractérisée en ce que** le prolongement (5) en forme de pivot est cylindrique dans la zone de la fente annulaire (14).

3. Tige de prothèse selon la revendication 1 ou 2, **caractérisée en ce que** le prolongement (5) en forme de pivot, sur son côté orienté vers la première partie (1), comporte un tronçon (6), qui se rétrécit en cône en direction de la fente annulaire (14) et qui est en appui contre un tronçon (13 ; 28) de la forure débouchante (4), lequel est conçu avec une forme complémentaire en s'élargissant en cône en direction de la première partie (1).

4. Tige de prothèse selon l'une quelconque des revendications précédentes, **caractérisée en ce que** la paroi intérieure (11, 12, 13) de la forure débouchante (4) est formée, dans la zone du prolongement (5) en forme de pivot, par un manchon (9) à face extérieure (10) cylindrique, qui entoure ledit prolongement et qui s'engage dans la forure débouchante (4) également cylindrique.

5. Tige de prothèse selon la revendication 4, **caractérisée en ce que** le manchon (9) comporte, au moins dans la zone de la fente annulaire (14), des encoches partant du bord parallèlement au sens axial.

6. Tige de prothèse selon l'une quelconque des revendications précédentes, **caractérisée en ce que** l'élément de serrage est un écrou de serrage (17) avec un taraudage (16), qui est vissé sur un tenon fileté (8) au niveau de l'extrémité du prolongement (5).

7. Tige de prothèse selon la revendication 6, **caractérisée en ce que** l'écrou de serrage (17), au niveau de son extrémité opposée à la fente annulaire (14), porte un tronçon de vissage (19), qui comporte des surfaces d'appui (22) pour un outil de vissage (23) et qui est relié à la partie restante de l'écrou de serrage (17) par l'intermédiaire d'une zone de rupture théorique (21).

8. Tige de prothèse selon la revendication 7, **caractérisée en ce que** les surfaces d'appui sont formées par un logement polygonal intérieur (22).

9. Tige de prothèse selon la revendication 7 ou 8, **caractérisée en ce que** la zone de rupture théorique (21) est agencée à hauteur de l'extrémité de la forure débouchante (4).

10. Tige de prothèse selon l'une quelconque des revendications 7 à 9, **caractérisée en ce que** dans l'écrou de serrage (17) est agencé un logement polygonal intérieur (22), qui comporte des surfaces d'appui pour un outil de dévissage et qui est accessible après le retrait du tronçon de vissage (19).

11. Tige de prothèse selon les revendications 8 et 10, **caractérisée en ce que** le logement polygonal intérieur (22) pour l'outil de dévissage est formé par un prolongement du logement polygonal intérieur (22) réalisé dans le tronçon de vissage (19).

12. Tige de prothèse selon l'une quelconque des revendications 7 à 11, **caractérisée en ce que**, entre le tronçon de vissage (19) et la partie restante (18) de l'écrou de serrage (17), est agencé un logement (20) en forme d'encoche pour des éléments de retenue (26) d'un outil de vissage (23), destinés à enserrer le tronçon de vissage (19).
